# EUROPEAN PATENT SPECIFICATION

(11) **EP 1 830 816 B1**
(45) Date of publication and mention of the grant of the patent: **01.08.2012**
(21) Application number: 05820824.0
(22) Date of filing: 20.12.2005
(51) Int. Cl.: A61K 9/16, A61K 31/4439, A61K 47/12, A61K 47/26, A61K 47/36, A61K 9/10, A61K 9/14

(54) **SOLID DOSAGE FORM COMPRISING PROTON PUMP INHIBITOR AND SUSPENSION MADE THEREOF**
FESTE DOSIERFORM MIT EINEM PROTONENPUMPENHEMMER UND DARAUS HERGESTELLTE SUSPENSION
FORME GALENIQUE SOLIDE COMPRENANT UN INHIBITEUR DE LA POMPE A PROTONS ET SUPENSION REALISEE A PARTIR DE LA LADITE GALENIQUE

(30) Priority: 22.12.2004 US 638435 P
(43) Date of publication of application: 12.09.2007
(73) Proprietor: AstraZeneca AB, 151 85 Södertälje (SE)
(72) Inventor: PERSSON, Eva, 221 87 Lund (SE); TROFAST, Eva, 221 87 Lund (SE)
(74) Representative: Wahlström, Stig Christer Gunnar
(86) International application number: PCT/SE2005/001972
(87) International publication number: WO 2006/068596

(56) References cited:
- EP-A1- 1 232 746
- WO-A1-00/74654
- WO-A1-2004/004718
- US-A- 5 731 002
- US-A1- 2004 170 684
- US-B1- 6 261 602
- FDA/Center for Drug Evaluation and ResearchOffice of Communications, Division of Information Services: "Prevacid (R) (lansoprazole)", , 5 March 2001 (2001-03-05), pages 4-28, XP002634923, Retrieved from the Internet: URL:http://www.accessdata.fda.gov/drugsatf da_docs/label/2001/21281lbl.pdf [retrieved on 2011-05-02]
- Product specification for Lanzo, 30 mg granules for oral suspension: ASPno: 1998-0084, NPL-id:19991007000057, Swedish Marketing: 14463, First approval date: 1999-10-07, Marketing Authorisation Holder: Wyeth Lederle Nordiska AB; product specification issued by the Swedisch Medical Products Agency ... XP002997521
- Product summary Produktresume for the above mentioned medical product Lanzo, in the form of granules for oral suspension; date for first approval for sale: 1999-10-07, date for review of the Product Summary: 2004-03-23; the Swedish Medical Products Agency (Lakemedelsverket);article in Swedish, 5 ... XP002997522

## Description

### Field of the invention

This invention relates to a solid rapidly gelling oral pharmaceutical dosage form, as well as aqueous suspensions prepared thereof, comprising an acid sensitive proton pump inhibitor as active ingredient distributed in a multitude of enteric coated pellets and a suspension modifying granulate. Furthermore the invention relates to an improved process for its manufacture and the use of such formulation in medical treatment including prevention of gastrointestinal disorders in humans.

### Background of the invention and prior art

Proton pump inhibitor (in the following also designated as "PPI") compounds having effect as H⁺K⁺-ATPase inhibitors are for instance compounds known under the generic names omeprazole, lansoprazole, pantoprazole, rabeprazole, tenatoprazole and esomeprazole.

These active substances are useful for inhibiting gastric acid secretion in mammals and man. In a more general sense, they may be used for prevention and treatment of gastric acid related diseases in mammals and man, including e.g. reflux esophagitis, gastritis, duodenitis, gastric ulcer and duodenal ulcer. Furthermore, they may be used for treatment of other gastrointestinal disorders where gastric acid inhibitory effect is desirable e.g. in patients on NSAID therapy, in patients with Non Ulcer Dyspepsia, in patients with symptomatic gastro-esophageal reflux disease, and in patients with gastrinomas. They may also be used in patients in intensive care situations, in patients with acute upper gastrointestinal bleeding, pre-and postoperatively to prevent acid aspiration of gastric acid and to prevent and treat stress ulceration. Further, they may be useful for prevention and treatment of irritable bowel syndrome (IBS), inflammatory bowel disease (IBD), ulcerative colitis, Crohn's disease, asthma, laryngitis, Barret's syndrome, sleep apnea, sleep disturbance, psoriasis as well as being useful for prevention and treatment of Helicobacter infections and diseases related to the above.

These active compounds are, however, susceptible to degradation/transformation in acidic and neutral media. The degradation is catalyzed by acidic compounds and is stabilized in mixtures with alkaline compounds. The stability of the active substances is also affected by moisture, heat, content of organic solvents and to some degree by light.

Oral dosage forms remain a significant problem for many patients, as many are unable or unwilling to swallow a solid dosage form. This problem occurs primarily in children and the elderly. It affects the patient compliance, and is therefore a problem in therapy.

The need for an oral administration form, which avoids the swallowing difficulties associated with traditional tablets, has been recognized since many years. Syrups, elixirs, microcapsules containing slurries and other novel tablet or capsule dosage forms have been developed. Among alternative forms for oral administration of pharmacologically active substances is the use of a solution or a suspension of the active ingredient in an aqueous medium.

Besides that ready to consume suspensions (or solutions) have drawbacks associated with larger storage volume and often limited shelf-life or need for refrigerator storage, a particular problem that sometimes arises with aqueous suspensions is that some solid particles have a strong tendency of sinking to the bottom of the vessel used for administration. This may cause a part of the dose to be retained in the vessel and not the entire dose entering the oral administration route. Another problem that is sometimes experienced, is when using a suspension of particles in a liquid medium for administration through a nasogastric tube, the particles may tend to aggregate or agglomerate, thereby making it impossble for them to pass through the used tube. Still another problem is when the liquid medium has a too high viscosity/viscoelasticity, which is making it impossible to administer it through a nasogastric tube at a practical pressure.

It is a strong desire, particularly when administering acid- labile compounds like proton pump inhibitors such as for instance omeprazole, esomeprazole, pantoprazole and lansoprazole, to get an easily and quickly prepared, easily swallowable homogeneous suspension comprising the proton pump inhibitor in a form protecting it from contact with acidic environments, (e.g. the acidic gastric fluids). In addition it is desirable that the suspension has viscoelastic properties and a viscosity suitable for allowing it to be administered via a gastric tube or to be swallowed. Also a liquid suspension formulation requires a certain viscosity to be stable over time.

With a drug preparation that is to be stored as a dry powder mixture comprising water unsoluble components, and which is intended to be given as an ex-tempore prepared homogeneous suspension, other challenges/problems arise.

For some prior art compositions there is a problem with that a maximum viscosity level is obtained only after long times, i.e. the viscosity is not constant over the short time frames from which the suspension is made until it usually is administered to the patient. There may also be problems with batch-to-batch variation regarding time required to obtain a stable maximum viscosity level in the suspension prepared from a dry powder mixture.

Intolerance to lactose-containing foods is a common problem. Thus, medicaments containing lactose may pose a problem for such people.

There are proposals in the art regarding compositions comprising a proton pump inhibitor and there are other proposals relating to methods for quickly dispersing and/or dissolving formulations.

US 5,731,002 describes a stable, oral pharmaceutical composition comprising a proton pump inhibitor in a paste-like gel designed for the treatment of gastric acid related diseases in animals.

US 5,840,737 discloses a method for treating gastric acid disorders with compositions comprising omeprazole or lansoprazole together with bicarbonates.
Problems associated with administering bicarbonates such as sodium- or potassium bicarbonate to i.a. humans include that when the carbonate is neutralized in the stomach belching may result. Patient with gastroesophageal reflux may exacerbate or worsen their disease as the belching can cause upward movement of stomach acid (Brunton, Agents for the control of gastric acidity and treatment of peptic ulcers. In: Goodman A G,et al. The pharmacologic basis of therapeutics, p. 907. (New York, 1990.)) Moreover, there is a possibility that intake of sodium bicarbonate may cause metabolic alkalosis.

Furthermore, there are further published patent applications in the same patent family, such US 2002/0045646 A1, which discloses a solid non-enteric coated dosage form compositions comprising a proton pump inhibitor and a buffer. Other applications in the family US2003/118669, US2003/144306, US2003/191159, US2003/215527, US2004/048896 and US2004/171646 disclose for instance liquid oral pharmaceutical compositions comprising a proton pump inhibitor and a buffering agent and method of increasing absorption of the proton pump inhibitor.

US 2004/0005362 A1 (Taneja) and US 2004/0082618 A1 (Taneja) describe a pharmaceutical formulation comprising an acid labile drug coated with an enteric coating and a liquid vehicle of pH less than 6.0. Other published applications from the same inventor describes for instance a liquid vehicle of a viscosity sufficient to suspend microgranules comprising a PPI (US 2004/0081700 A1) or (US 2004/0006109 A1 and US 2004/0081671 A1) principally the same arrangement wherein the pH of the liquid vehicle is greater than 6.5.

WO 2004/004690 A1 (Taneja) discloses a liquid dosage form having enteric coated microgranules comprising an acid-labile drug and a liquid suspension having a pH less than 6.0 and a viscosity sufficient to suspend the microgranules. Carbonates or bicarbonates may be used in the dosage forms.

US 2004/0022854 A1 discloses an oral administration form for acid-labile active compounds wherein the auxiliaries are not suitable for formation of enteric layers (enteric coating). Prepared active compound units can be formulated into sachets, e.g together with lactose, or formulated together with carbonate containing excipients to an effervescent composition.

EP 1,232,746 describes a readily suspendible dry powder mixture composition comprising a gellant or thickener, comprising at least one xanthan gum having a specific particle size distribution, a filler, a wetting agent or surfactant, and a pharmacologically active substance.

US 4,886,669 describes a water-dispersible tablet comprising a pharmaceutically active agent, at least one disintegrant and a swellable material. It is stated that the tablet disintegrates rapidly in water forming a homogeneous suspension of high viscosity that can easily be swallowed.

US 5008117 relates to a method for preparing a quickly dispersing and dissolving formulation of thickening or suspending agents and other excipients, in which drug microcapsules are readily dispersible. Proton pump inhibitors are not mentioned.

EP 0491910 B1 discloses a solid pharmaceutical composition for addition to water to produce a suspension of a drug. The composition comprises a thickening or suspending agent, an acid, and a carbonate or bicarbonate.

US 6,261,602 describes a granular composition useful as a pharmaceutical carrier which can be used for the preparation of pharmaceutical compositions that are capable of rapid suspension in water or aqueous media. The composition may be prepared by a process which comprises subjecting a mixture of a thickening agent and a disintegrating agent to wet granulation with an aqueous medium as wetting agent or dry granulation to make a granular product.

### Brief description of the invention

The present invention avoids the above discussed disadvantages with prior art compositions and presents a solution to the previous mentioned problems. It further provides a mean for making a drug vehicle, which is suitable for administration via a gastric tube, due to good viscosity and viscoelasticity properties of the obtained vehicle (suspension). For instance, in the sense that it is e.g. robust enough to provide approximately the same viscosity even if the used water amount varies within 50% to 150% of the prescribed amount.

The present invention relates to a solid rapidly gelling oral pharmaceutical dosage form comprising an acid sensitive proton pump inhibitor compound as active ingredient distributed in a multitude of enteric coated pellets and a suspension modifying granulate.

Furthermore, it has now surprisingly been found that it is advantegeous to use a special composed granulate to mix with a multitude of enteric coated pellets comprising a proton pump inhibitor, which granulate when suspended in water, quickly and reproducible will create an aqueous vehicle having a desired pH, a desired, stable viscosity level and a satisfying viscoelasticity. This granulate is in the following also referred to as "suspension modifying granulate". Furthermore, this granulate should be free from bicarbonate and carbonate salts. According to one enbodiment of the invention, it is possible to make this granulate free from lactose, i.e. tolerable for people having intolerance to lactose.

The dosage forms of the invention render the quick formation of a viscous stable suspension possible. Prior to administration, the solid dry suspension modifying granulate and the enteric coated pellet are dissolved/suspended in an aqueous liquid, such as tap water providing a viscous liquid formulation for oral administration. When a dosage form of the invention is to be administered to the patient, it is important that the preparation will be dissolved/suspended as fast as possible and at the same time provides a homogeneous suspension with regard to the distribution of the solid particles containing the pharmacologically active ingredient. Therefore, the final liquid formulation should secure that practically all of the dose, even if the dose is comprised in a suspended, particulate form, is delivered into the oral cavity, i.e. the entry of the oral administration route, in a safe, reliable and reproducible way.

When the active ingredient is comprised in enteric coated pellets, it is necessary that the suspension medium has a pH that does not cause premature dissolution of the enteric coating layer of the pellets comprising the active ingredient. Also the administration through naso-gastric tubes puts demands on the final liquid formulation regarding such things as suitable and stable viscosity, viscoelastic properties and absence of agglomeration tendencies of the suspended particles.

A further feature is that the suspension is suitable for administration with thin tubes aimed for pediatric use. The expression gastric tube includes naso-gastric tubes as well as other tubes or syringes aimed for feeding a suspension or dispersion into the stomach of a patient.

The viscoelastic and viscosity properties become especially important as tubes used in pediatric treatment may have a narrow inner diameter and thereby being sensitive for liquids having unsuitable properties giving high back-pressures upon administration. One such example of a tube with narrow inner diameter is "Infant feeding tube, FT 1606/105 (CH/FG 6 -2.0 mm outer diameter, 1.4 mm inner diameter), Pennine Healthcare."

The dosage forms of the invention are gelling quicker in water at room temperature than prior art formulations to yield a homogeneous stable dispersion. Thus, they give a stable viscosity in shorter time than the prior art, and furthermore they are robust in respect of obtained viscosity properties.

In brief, the dosage forms of the invention comprise two principal components; a suspension modifying granulate and a multitude of enteric coated pellets comprising the active ingredient.

The suspension modifying granulate comprises;
- a rapidly dissolving diluent
- a gelling agent
- an acidic pH-regulating agent
- a binder and
- an optional disintegrant, and
furthermore, the granulate is free from bicarbonate salts and/or carbonate salts.

According to one feature, the above described suspension modifying granulate is free from lactose. This further advantage makes it suitable for people suffering from lactose-intolerance and they can be treated with embodiments of the invention.

One of the features of the invention is that the rapidly dissolving diluent is brought into close/intimate contact with the gelling agent. This does not only give a very rapid gelling time compared to the gelling agent per se, but also very quickly a stable gel. The selection of an accurately diluent, which also may function as a sweetener, is one embodiment of the invention.

According to one feature of the invention, the rapid disintegration and quick gelling to a stable and reproducible viscosity level when the suspension modifying granulate is suspended in water, is achieved by a special manufacturing process. According to this feature the process comprises that the gelling agent and diluent/sweetener are mixed and granulated together and thereafter dried to obtain a low moisture and/or solvent content. Manufacture of the enteric coated pellets is described in the section "Detailed description of the invention", but they can in general be manufactured according to directives in WO 9601624 A1, with consideration taken to the special desires of size. Furthermore, there is no need for any "overcoat" on the enteric coated pellets.

The present invention provide a safe and reliable dosage forms for administration of enteric coated pellets comprising acid-labile proton pump inhibitors such as omeprazole, esomeprazole, pantoprazole and lansoprazole dispersed in an aqueous liquid medium, and also through gastric tubes. This is especially suitable and advantageous in the treatment of geriatrics or pediatrics.

The compositions of the present invention also allow the incorporation of a wide range of dosage levels and additional agents like taste masking/improving agents and tonicity agents.

### Brief description of the Figures

Figure 1 shows viscosity versus time for an embodiment of the invention. (5 samples)
Figure 2 shows viscosity versus time for a prior art embodiment (Lanzo^{™}, 4 samples).

### Detailed description of the invention

An oral pharmaceutical dosage form being a solid rapidly gelling granulate mixture, suitable for making a suspension comprising I) an acid sensitive proton pump inhibitor being esomeprazole, an alkaline salt thereof or a hydrated form of anyone of them as active ingredient distributed in a multitude of enteric coated pellets and II) a granulate, characterized in that the granulate is a suspension modifying granulate comprising a rapidly dissolving diluent selected from glucose and sucrose and hydrates of either of them, a gelling agent chosen among xanthan gums, an acidic pH-regulating agent, a binder and an optional disintegrant and the granulate is free from bicarbonate salts and/or carbonate salts and wherein the ratio between the binder and the gelling agent in the granulate (II) is from 1:2 to 1:3, w/w.

One aspect of the present invention is a dosage form being a mixture of one component (I) being a multitude of enteric coated pellets and another component (II) being a suspension modifying granulate, the mixture being dispensed in a container like e.g. a sachet. The mixture is rapidly disintegrating and gelling when suspended in an aqueous medium, such as tap water, thus forming a homogeneous stable and robust suspension having a

reproducible and stable viscosity, a suspension that can easily be swallowed or administered through e.g. a naso-gastric tube, by the patient. The liquid formulation ready to use is a further aspect of the present invention, i.e. it comprises three components, the two components mentioned above (I) and (II) and in addition the liquid medium (III).

The rapid gelling, i.e. the short gelling time obtained, of the present invention, can i.a. be seen as an effect on the time required before substantially all of the enteric coated pellets in the prepared suspension remain suspended in the liquid medium and not sinking to the bottom of the vessel (glass, beaker), used for its preparation. The gelling time required for embodiments of the invention is in general shorter than 3 minutes, and preferably less than 2 minutes, when tested as described in Example 5.

The dosage form is free from bicarbonate salts and/or carbonate salts. One embodiment of the invention is furthermore free from lactose. "Free from" means that no such compound is added in the formulation. Trace amounts present in and accompanying other raw materials used in the composition are not taken into account by this expression.

### Enteric coated pellets

The enteric coated pellets comprising the active ingredient are manufactured with the outermost layer being the enteric coating layer. Such pellets can be manufactured according to methods known in the art, e.g. as described in WO 9601624 A1, taking into consideration the special desires on size of the pellets. Furthermore, there is no need for any "overcoat" on the prepared enteric coated pellets.

According to one aspect of the invention, the average diameter of the enteric coated pellets is 0.2 - 1.8 mm in diameter, preferably 0.4 - 1.0 mm in diameter and more preferably 0.5-0.8 mm in diameter.

In another aspect of the invention the enteric coated pellets are in the size range of 1.0-1.4 mm in diameter.

The enteric coated pellets are consisting of the following structural components;
- a core material comprising the active ingredient,
- an optional separating or subcoating layer, and
- an enteric coating layer,
but no additional coating layer on the enteric coating layer.

### Core material.

Core material is manufactured by processes known in the art such as extrusion-spheronization, layering techniques such as powder- or solution/suspension layering, spray drying, balling, congealing techniques or spray congealing techniques.

The core material comprises the active ingredient and may also comprise seeds, binders, surfactants, fillers, disintegrating agents, alkaline additives or other pharmaceutically acceptable ingredients, alone or in mixtures.

### Active ingredient

The pharmaceutical formulations of the invention comprise an acid sensitive proton pump inhibitor being esomeprazole, an alkaline salt thereof or a hydrated form of any of them as actiove ingredient.

The active ingredient is being comprised optionally together with excipients, in small enteric coated pellets/beads.

The compound for the oral pharmaceutical preparation according to the present invention is the (-)-enantiomer of omeprazole. The latter having the generic name esomeprazole. According to one embodiment the active ingredient is esomeprazole magnesium trihydrate.

According to another aspect of the invention the compound/ active ingredient is a hydrated form of anyone of the aforementioned compounds/active ingredients.

In one aspect of the invention, the amount of active ingredient in the preparation is in the range of 1 mg - 100 mg, 2 mg - 80 mg or 5 mg - 50 mg.

### Seeds

The seeds, which are to be layered with the active substance, can be water insoluble seeds comprising different oxides, celluloses, organic polymers and other materials, alone or in mixtures or water soluble seeds comprising different inorganic salts, sugars (excluding lactose), non-pareils and other materials, alone or in mixtures. Further, the seeds may comprise active substance in the form of agglomerates, compacts etc.

### Binders

Binders are for example celluloses such as hydroxypropyl methylcellulose, hydroxypropyl cellulose and carboxymethyl-cellulose sodium, polyvinyl pyrrolidone, polyethylene glycols, polyvinyl alcohols, sugars (excluding lactose), starches and other pharmaceutically acceptable substances with cohesive properties.

### Surfactants

Surfactants may be used in the dosage form. Suitable surfactants are found in the groups of pharmaceutically acceptable non-ionic surfactants such as for instance Polysorbate 80 or ionic surfactants such as for instance sodium lauryl sulfate.

### Fillers

Fillers may be used in the dosage form. Examples of fillers include for instance mannitol and dicalcium phosphate.

### Disintegrating agents

Disintegrating agent may be used in the dosage form. Examples of disintegrating agents that can be used are for instance cross-linked polyvinyl pyrrolidone, pregelatinized starch, microcrystalline cellulose, and cross-linked sodium carboxymethyl cellulose.

### Alkaline additives

According to one embodiment of the invention, the active substance may also be mixed with an alkaline pharmaceutically acceptable substance (or substances). Such substances can after excluding bicarbonate salts or carbonate salts be chosen among, but are not restricted to, substances such as the sodium, potassium, calcium, magnesium and aluminium salts of phosphoric acid, citric acid or other suitable weak inorganic or organic acids; substances normally used in antacid preparations such as aluminium, calcium and magnesium hydroxides; magnesium oxide; organic pH-buffering substances such as trihydroxymethylaminomethane, basic amines or amino acids and their salts or other similar, pharmaceutically acceptable pH-buffering substances.

### Separating or subcoating layer

The separating or subcoating layer(s) can be applied to the core material by coating or layering procedures in suitable equipments such as coating pan, coating granulator or in a fluidized bed apparatus using water and/or organic solvents for the coating process. As an alternative the separating layer(s) can be applied to the core material by using powder coating technique. The materials for separating layers are pharmaceutically acceptable compounds such as, for instance, sugar, polyethylene glycol, polyvinylpyrrolidone, polyvinyl alcohol, polyvinyl acetate, hydroxypropyl cellulose, methyl-cellulose, ethylcellulose, hydroxypropyl methyl cellulose, carboxymethylcellulose sodium and others, used alone or in mixtures. Additives such as plasticizers, colorants, pigments, fillers, anti-tacking and anti-static agents, such as for instance magnesium stearate, titanium dioxide, fumed silica, talc and other additives may also be included into the separating layer(s).

The separating layer(s) may serve as a diffusion barrier and may act as a pH-buffering zone. The pH-buffering properties of the separating layer(s) can be further strengthened by introducing into the layer(s) substances, after excluding bicarbonate salts or carbonate salts,chosen from a group of compounds usually used in antacid formulations such as, for instance, magnesium oxide, hydroxide, aluminium or calcium hydroxide or silicate; composite aluminium/magnesium compounds such as, for instance MgO.Al₂O₃.2SiO₂.nH₂O, or other pharmaceutically acceptable pH-buffering compounds such as, for instance the sodium, potassium, calcium, magnesium and aluminium salts of phosphoric, citric or other suitable, weak, inorganic or organic acids; or suitable organic bases, including basic amino acids or amines and salts thereof. Talc or other compounds may be added to increase the thickness of the layer(s) and thereby strenghten the diffusion barrier.

### Enteric coating layer

One or more enteric coating layers are applied onto the core material or onto the core material covered with separating layer(s) by using a suitable coating technique. The enteric coating layer material may be dispersed or dissolved in either water or in suitable organic solvents. As enteric coating layer polymers one or more, separately or in combination, of the following can be used; e.g. solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s).

The enteric coating layers contain pharmaceutically acceptable plasticizers to obtain the desired mechanical properties, such as flexibility and hardness of the enteric coating layers. Such plasticizers are for instance, but not restricted to, triacetin, citric acid esters, phthalic acid esters, dibutyl sebacate, cetyl alcohol, polyethylene glycols, polysorbates or other plasticizers.

### Suspension modifying granulate.

The suspension modifying granulate comprises;
- a rapidly dissolving diluent,
- a gelling agent,
- an acidic pH-regulating agent,
- a binder, and
- an optional disintegrant
and in addition it is free from bicarbonate salts or carbonate salts, especially components that can result in effervescense.

According to one embodiment, the suspension modifying granulate is manufactured by a process in which the rapidly dissolving diluent and the gelling agent are mixed and granulated together, and thereafter dried.
The final moisture content in the suspension modifying granulate measured as loss on drying is < 3 % (w/w), preferably < 1 % (w/w). The final content of ethanol is < 0.2 % (w/w), preferably less than 0.12 % (w/w).

When the suspension modifying granulate is suspended in tap water, a stable and close to maximum viscosity is obtained in a short time. Further, the suspension obtained is free from lumps and is robust, in the sense that its viscosity properties are approximately the same even if a patient adds too little or too much water when preparing the suspension from the granulate. Thus, it is possible to add one dose of the active ingredient and the suspension modifying granulate to from 50% up to 150% of the prescribed amount of water and still obtain the desired properties of the formulation.

The gel formed when adding the suspension modifying granulate to an aqueous medium, such as water, has a viscosity of 3.0 to 6.0 log (mPas) = 10³ to 10⁶ mPas, preferably 3.6 to 4.7 log (mPas) = 10^{3.6} to 10^{4.7} mPas.
This viscosity is evaluated at 20°C from the intercept at the viscosity axis of the line when plotting log (viscocity) against log (rotational speed (rpm)). The line is made by a linear fit using least square linear regression and the intercept of the fitted line is determined. Suitable equipment for determination of viscosity is used, such as a Physica DV1 P viscometer, measuring geometry is No. 2 spindle, 18.7 mm in diameter, length 6.9 mm, which is operated at rotational speed 3.0, 6.0, 30 and 100 rpm, and measurements are made until a stable value is obtained (about 1 minute).

The rapid disintegration and quick gelling to a stable and reproducible viscosity level when the suspension modifying granulate is suspended in water is achieved, according to one aspect of the invention when a special manufacturing process for preparing the suspension modifying granulate is used.

This manufacturing process including the following steps in the following order not excluding the alternative that steps I and II can be interchanged;
I) mixing the gelling agent with the pH-regulating agent, the rapidly dissolving diluent and the optional disintegrant
II) dissolving the binder in ethanol
III) wetting the mixture obtained in step I (alternatively in step II if the order is interchanged) with the solution obtained in step II (alternatively in step I if the order is interchanged)
IV) agitating the wet mixture obtained in step III in order to have almost each particle of the gelling agent to be in close/intimate contact with the above mentioned rapidly dissolving diluent
V) drying the agitated wet mixture from step IV until the final moisture content in the suspension modifying granulate measured as loss on drying is < 3 % (w/w), preferably < 1 % (w/w)
VI) grinding or milling the dry granules obtained in step V until more than 95% (w/w) of the granules passes a sieve having 1.0 mm openings.

One feature of the invention is to bring the rapidly dissolving diluent into close/intimate contact with the gelling agent, thereby not only giving a very rapid gelling time compared to the gelling agent per se, but also very quickly a stable gel. One embodiment of the invention is the selection of a suitable rapidly disintegrating diluent, which also may function as a sweetener.

The rapid gelling of the dry suspension modifying granulate in general when added to water, such as tap water, is seen as that gelling is generally sufficient, i.e. reaching 75% of the maximum obtainable level, already within approx. 10 minutes. 90% or more of the maximum viscosity is generally reached within 15 minutes. For comparison see the Table in Example 2.

Specifically, when a single suspension modifying granulate according to the invention is suspended in water and mildly agitated it gives a suspension that reaches more than 75 % of the maximum obtainable viscosity within 13 minutes, preferably more than 75 % within 10 minutes, tested with 1 g of suspension modifying granulate added to 5 ml of water. More than 90 % of the maximum obtainable viscocity is reached within 30 minutes, preferably more than 90 % within 25 minutes, tested with 1 g of suspension modifying granulate added to 5 ml of water.

According to one embodiment of the invention the suspension modifying granulate (and the enteric coated PPI comprising pellets) does not contain lactose.

### Gelling agent

The gelling agent provides for forming a gel suitable for administration through a gastric sond/ naso-gastric tube, i.e. chosen to have the proper viscoelasticity as well as the proper viscosity of the gel formed when dispersed in an aqueous medium, such as water. This is a desired administration route in paediatric or geriatric therapy.

The dissolution time will also influence the selection of gelling agents.

Suitable gelling agents of the invention are different qualities of xanthan gums.

Gelling agents like Na carboxymethylcellulose (CMC) and carrageenan could not be used in the present invention due to lack of suitable viscoelastic properties or due to unsuitable properties for administering the obtained suspension through a gastric sond.

Thus, gelling agents in the invention are chosen among xanthan gums.

The concentration of the gelling agent is 0.6 to 12 % w/w of the suspension modifying granulate. In a preferred embodiment the concentration of the gelling agent is between 1.8 to 4.8 % w/w. It is suitable to have such a broad range in concentration of the gelling agent of practical reasons for the patient and still having suitable properties of the viscoelestic gel.

In one embodiment of the invention the manufacturing is performed with the gelling agent having an average particle size larger than 150 microns.

### Rapidly dissolving diluent

The diluent has a diluting function but it may also function as a sweetener.
The diluent is selected from the group consisting of glucose and sucrose and hydrates of either of them.

With rapid dissolution is ment, according to the present invention, that the dissolution time of the diluent is below 2 minutes when 2 g of the substance is dissolved in 10 ml of water during slow continous stirring at 14°C. One diluent specifically not fullfilling this requirement is mannitol.

As a consequense of the manufacturing method, the suspension modifying granulate according to the invention has the rapidly dissolving diluent randomely distributed in and on the obtained individual granule particles.

### Acidic pH-regulating agent

The suspension modifying granulate when suspended in water forms a suspension having a pH in the range from 3.0 to 6.0, preferably in the range from 3.0 to 5.0, and more preferably in the range from 3.5 to 4.5.

This may be achieved by adding a suitable acidic pH-regulating agent. This agent may consist of a single acidic chemical compound or a mixture of compounds chosen among acidic and alkaline compounds, with the exception of any carbonate salts. Any mixture of such pH influencing compounds is chosen in such a way that when the mixture is dissolved/suspended in water it will give a pH within the desired (acidic) range according to above.

Non-limiting examples of suitable acidic compounds are; Citric acid, tartaric acid and malic acid. Non limiting example of a mixture of compounds chosen among acidic and alkaline compounds is monosodiumphosphate and disodium phosphate (in appropriate ratio to achieve a pH within the desired range).

### Disintegrant

The optional disintegrant used in the dry suspension modifying granulate may be a single disintegrant or a mixture thereof.

Non-limiting example of suitable disintegrants include: Polyvinyl pyrrolidone cross-linked, crosslinked Sodium carboxymethyl cellulose (Ac-Di-Sol^{®}) and pregelatinized starch (Sta-Rx^{®} 1500).

### Binder

A suitable binder used according to the present invention is a polymer that is soluble in water and in ethanol. Suitable ones are selected qualities of hydroxypropylcellulose. When the binder is chosen to be a hydroxypropyl cellulose (in the following also referred to as HPC), it is having a hydroxypropyl content in the range of 50 - 90%, or more preferably in the range of 60 - 81%, and a viscosity below 450 mPas (cps) tested at 5% concentration. Such a polymers are, example given, Klucel^{®} JF and Klucel^{®} LF from Aqualon.

The hydroxypropyl celluloses contemplated for use in this aspect of the invention, as a binder, do not include Low-substituted hydroxypropyl cellulose, also preferred to as L-HPC.

The ratio between the binder and the gelling agent in the suspension modifying granulate of the invention is from 1:2 to 1:3, w/w.

### Dosage form strengths

Different product strengths are obtained by filling specific amounts of enteric coated proton pump inhibitor pellets and the suspension modifying granulate of the invention into unit size sachets. According to one embodiment of the invention the enteric coated pellets comprising esomeprazole magnesium trihydrate are filled together with the suspension modifying granulate into unit size sachets.

The ratio w/w between the two components of the mixture - i.e. between the proton pump inhibitor comprising enteric coated pellets on the one hand and the (dry) suspension modifying granulate on the other hand - may vary between 1: 1000 to 100 : 1000, preferably between 4 : 1000 to 80 : 1000 and most preferably between 8 : 1000 to 60 : 1000.

### Amount of enteric coated pellets in one sachet.

The PPI comprising enteric coated pellets have a drug content from 5% w/w of the enteric coated pellets to 40% w/w of the enteric coated pellets. This means that the highest theoretical amount of pellets for one dose can be calculated considering the situation when the lowest concentration of drug in pellets is at hand for the highest dose of drug (100 mg acc. to invention) giving a total of (100/0.05=) 2000 mg pellets.

The lowest possible amount of pellets can in an analogue reasoning for the opposite situation be calculated from the highest concentration and the lowest dose (1 mg acc. to the invention) gives the minimum amount of pellets to (1/0.4=) 2.5 mg pellets.

In a preferred embodiment of the invention the drug content of the enteric coated pellets is 8 - 30 % w/w.

The amount of enteric coated pellets in one sachet according to the invention is in the range of 2.5 - 2000 mg, and in the preferred embodiment of the invention the amount of enteric coated pellets in one sachet is in the range of 3 - 1250 mg.

In an alternative embodiment of the invention, the drug content of the enteric coated pellets are adapted to the dose of drug intended in one sachet, according to the following table;

**Table 1.**

| Dose in one sachet intended | Adapted drug content in the enteric coated pellets | Amount of pellets in one sachet |
|---|---|---|
| 1 mg - 40 mg | 8-12 % w/w | 8 - 500 mg |
| > 40 mg - 70mg | 15-25 % w/w | 160 - 467 mg |
| > 70 mg - 100 mg | 25 - 40 % w/w | 280 - 400 mg |

Thus in one embodiment of the invention the dose in one sachet is 1-40 mg and the drug content in the enteric coated pellets is 8-12 % (w/w).

In another embodiment of the invention the dose in one sachet is >40 mg - 70 mg and the drug content in the enteric coated pellets is 15-25 % (w/w).

In a further embodiment of the invention the dose in one sachet is >70 mg - 100 mg and the drug content in the enteric coated pellets is 25-40 % (w/w).

### Liquid formulation ready for use

Prior to use the content of the sachet is emptied into a predefined volume of aqueous liquid. After stirring, a viscous suspension is formed. This liquid formulation is another aspect of the invention and is comprising three main components being enteric coated pellets comprising proton pump inhibitor , (dry) suspension modifying granulate and aqueous liquid.

The amount of aqueous liquid is aimed to be 5 times the amount of suspension modifying granulate, but allowing for the patient to vary this liquid amount from 50 % up to 150% of the prescribed amount. This means that the aqueous liquid amount in the liquid formulation ready for use is from 2.5 times up to 7.5 times the amount of the suspension modifying granulate.

In one aspect of the invention the aqueous liquid is water.

The concentration of the gelling agent should be 0.1 to 2 % w/w (a twenty fold range in concentration) of the suspension, preferably between 0.3 to 0.8 % w/w. It is advantages to have such a broad range in concentration of the gelling agent of practical reasons for the patient and still maintaining relevant properties of the viscoelestic gel.

### Examples

### Example 1a.

### Preparation of the suspension modifying granulate according to the invention

| Excipient | Content (%) |
|---|---|
| Xanthan Gum 11K | 2.5 |
| Polyvinylpyrrolidone cross-linked | 2.5 |
| Glucose, water free | 93.8 |
| Hydroxypropyl cellulose JF | 1.0 |
| Citric acid anhydrous | 0.164 |
| Colour iron dioxide yellow | 0.06 |

The hydroxypropyl cellulose is dissolved in ethanol. The solution is added to a dry mixture of the remaining excipients giving a wet mass and the mass being granulated during the addition of the solution. The wet mass is dried and grinded (maximum 5 % of the granules > 1 mm).

3 g of this suspension modifying granulate was dissolved in 15 ml water and the liquid formulation was stirred for 60 s. The pH was measured with a glass electrod using a calibrated pH meter and found to be 4.0.

### (Comparative) Example 1b.

### Suspension modifying granulate according to prior art

As comparison has been used the commercially product "Lanzo^{™} 30mg, granulate" from Wyeth Lederle (batch 3ET032, expiry date July 2006 and 3ET010, expiry date March 2006.

The suspension granulate composition (excluding the enteric coated pellets) used for this product is according to SWEDIS:

| Excipient | Content (%) |
|---|---|
| Mannitol | 45.8 |
| Sucrose | 45.8 |
| Xanthan gum | 3.5 |
| Polyvinylpyrrolidone, cross-linked | 3.5 |
| Dioctyl sulfosuccinate | 0.015 |
| Magnesium stearate | 0.5 |
| Silicon dioxide | 0.1 |
| Citric acid anhydrous | 0.4 |
| Color | 0.05 |
| Flavouring | 0.4 |

### Ex 2. Viscosity measurements.

### - experimental conditions:

Embodiment according to the inventon: 3 g suspension modifying granulate obtained according to Example 1a was dissolved in 15 ml water and the liquid formulation was stirred for 60 s. TM

Prior art sample (Lanzo 30 mg, granulate): The Lansoprazole comprising pellets were removed from the total solids (5.7 g) of the product described in Example 1b and to the remaining powder/granulate (5.4 g) was added 30 ml water whereafter the liquid formulation was stirred for 60 s.

For both samples viscosity measurements started after another 1 min.
Instrument: Reologica Stresstech
Measuring principle: Oscillation with plate/plate P 30 2 mm slit
Measuring parameters: Frequency 0.1 Hz; stress 0.07146 Pa.

| Time to arrive at Viscosity in % of Maximum Viscosity (Evaluated from Figure 1 and 2.) | | |
|---|---|---|
| Percentage of maximum Viscosity | > 75% | > 90% |
| Ex. 1a (invention) n=5 | average = 9.7 min | average = 14.8 min |
| | min= 7.6 min | min= 9.5 min |
| | max= 12.6 min | max= 23.1 min |
| Ex. 1b (prior art) n=4 | average = 16.8 min | average = 32.5 min |
| | min= 13.3 min | min= 29.0 min |
| | max= 21.2 min | max= 39.5 min |

### Discussion

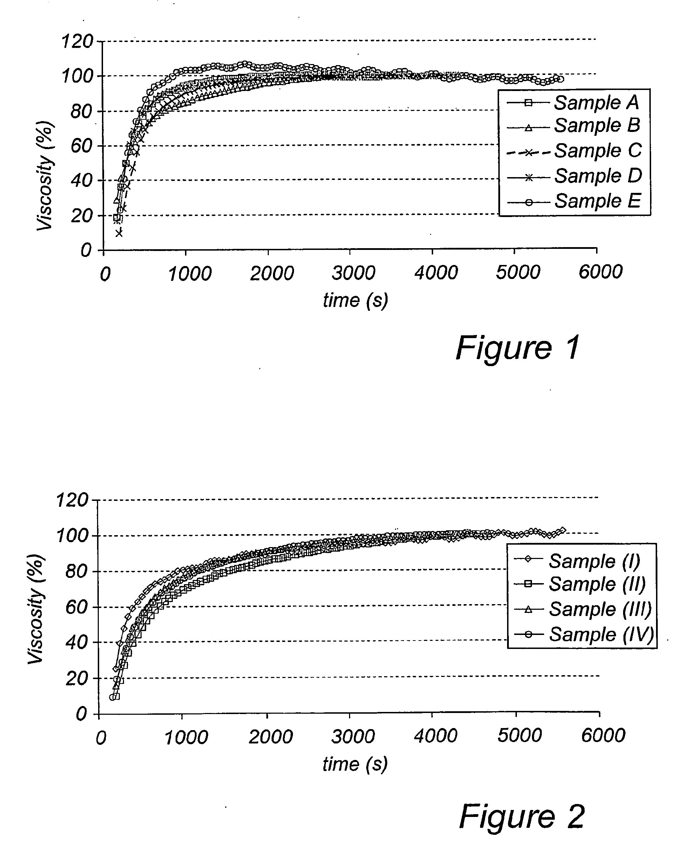

In the case of lansoprazole (Ex. 1b), although having a fast dissolving diluent (sucrose) being added to the suspension granulate formulation, the formulation will not form a stable gel within the desired shorter time frame, see Figure 2, to be compare with that which is obtained with the present invention (Ex. 1a), see Figure 1, and the results shown in the Table above.

The result of using a slow dissolving diluent will be a composition with slower gelling and a continous increasing viscosity within a reasonable and adequate time period. Thus, the present invention has solved several problems in order to obtain a lactos- free and bicarbonate/carbonate-free composition having rapid gelling time with a viscosity/viscoelasticity suitable for swallowing or administration through a tube, such as constant viscosity over time, and no lumps present in the final suspension to be administered.

### Example 3. Manufacturing of enteric coated pellets comprising esomeprazole-Mg-trihydrate.

| Core material | |
|---|---|
| Esomeprazole-Mg trihydrate | 445 g |
| Sugar sphere seeds | 300 g |
| Hydroxypropyl methylcellulose | 67 g |
| Polysorbate 80 | 9 g |
| Purified water | 2100 g |

| Subcoating layer | |
|---|---|
| Hydroxypropyl cellulose | 90 g |
| Talc | 340 g |
| Magnesium stearate | 22 g |
| Purified water | 3100 g |

| Enteric coating layer | |
|---|---|
| Methacrylic acid copolymer type C, 30 % dispersion | 1270 g |
| Triethyl citrate | 38 g |
| Mono- and diglycerides | 19 g |
| Polysorbate 80 | 2 g |
| Purified water | 500 g |

Suspension layering was performed in a fluid bed apparatus using bottom spray technique. Esomeprazole was sprayed onto the sugar sphere seeds from a water suspension containing the dissolved binder and surfactant. The size of the sugar spheres seeds were in the range of 0.25 to 0.35 mm.

The prepared core material was covered with the subcoating layer in a fluid bed apparatus with a hydroxypropyl cellulose solution containing talc and magnesium stearate. The enteric coating layer was sprayed as a water dispersion onto the pellets covered with separating layer in a fluid bed apparatus.

### Example 4. Examples of component ratios for preparing final liquid formulation of different dose strength.

| | Strength (amount of active drug, e.g.esomeprazole, per sachet) | | |
|---|---|---|---|
| | 2.5 mg | 10 mg | 40 mg |
| Amount of enteric coated. pellets*, (in sachet) (mg) | 10.6 | 42.6 | 170 |
| Amount of suspension modifying granulate ** (in same sachet as above) (g) | 1 | 3 | 3 |
| Volume of water (ml) | 5 | 15 | 15 |

| | | | |
|---|---|---|---|
| * made in accordance with Example 3. ** made in accordance with Example 1a. | | | |

### Example 5. Illustration of the rapid gelling time of the present invention.

The content of a sachet containing the final formulation, of 40 mg dose strength according to Ex.4 was emptied into a beaker containing the nominally prescribed 15 ml amount of water.

Then the sample was stirred for 15 seconds and then kept resting until 55 seconds from start. After that it was again stirred for 5 seconds, to evenly distribute the active drug granules in the suspension.

Then the suspension was inspected for 30 seconds to determine whether substantially all of the enteric coated pellets were distributed in the suspension or if they were assembled at the bottom of the beaker.

If the pellets were not distributed in the liquid medium but assembled at the bottom of the beaker, the process was repeated, i.e. meaning waiting 25 seconds further and stirring 5 seconds ,i.e. until 2 minutes, followed by inspecting for 30 seconds, until substantially all of the pellets remained distributed in the liquid medium. The time needed for the pellets to be remaining in the liquid medium was recorded.

The samples in the table below were evaluated in the described way, with the following results;

| **Sample** | **Time needed for pellets to remain suspended** |
|---|---|
| 1) Sachet cont. 40mg dose strength, acc. to Ex. 4. | 2 minutes |
| 2) Sachet cont. 40mg dose strength, acc. to Ex. 4. | 2 minutes |
| 2) Sachet cont. 10mg dose strength, acc. to Ex. 4. | 2 minutes |
| | |
| I) Sachet "Lanzo^{™} 30 mg" | 5 minutes |
| II) Sachet "Lanzo^{™} 30 mg" | 5 minutes |

## Claims

1. An oral pharmaceutical dosage form being a solid rapidly gelling granulate mixture, suitable for making a suspension comprising I) an acid sensitive proton pump inhibitor being esomeprazole, an alkaline salt thereof or a hydrated form of anyone of them as active ingredient distributed in a multitude of enteric coated pellets and II) a granulate, **characterized in that** the granulate is a suspension modifying granulate comprising a rapidly dissolving diluent selected from glucose and sucrose and hydrates of either of them, a gelling agent chosen among xanthan gums, an acidic pH-regulating agent, a binder and an optional disintegrant and the granulate is free from bicarbonate salts and/or carbonate salts and wherein the ratio between the binder and the gelling agent in the granulate (II) is from 1:2 to 1:3, w/w.

2. The dosage form according to claim 1, which is free from lactose.

3. The dosage form according to any of claims 1 to 2, wherein the rapidly dissolving diluent and the gelling agent are mixed and granulated together such that the rapidly dissolving diluent is randomly distributed in and on the obtained granule particles.

4. The dosage form according to any of claims 1 to 3, wherein the concentration of the gelling agent is 0.6% to 12% w/w of the suspension modifying granulate (II).

5. The dosage form according to any of claims 1 to 3, wherein the concentration of the gelling agent is 1.8% to 4.8 % w/w of the suspension modifying (granulate (II).

6. The dosage form according to any of claims 1 to 5, wherein the suspension modifying granulate (II) when suspended in water forms a suspension having a pH in the range from 3.0 to 6.0.

7. The dosage form according to any of claims 1 to 5, wherein the suspension modifying granulate (II) when suspended in water forms a suspension having a pH in the range from 3.0 to 5.0.

8. The dosage form according to any of claims 1 to 7, wherein the enteric coated pellets are consisting of the structural components; a core material comprising the active ingredient, a subcoating layer, an enteric coating layer and no additional coating layer on the enteric coat

9. The dosage form according to any of claims 1 to 8, wherein the enteric coated pellets have an average diameter of 0.2 - 1.8 mm in diameter.

10. The dosage form according to any of claims 1 to 8, wherein the enteric coated pellets have an average diameter of 0.4 - 1.0 mm in diameter.

11. A sachet comprising the dosage form according to any of claims 1-10.

12. A sachet according to claim 11, wherein the amount of active ingredient is 1 mg - 100 mg.

13. A sachet according to claim 11, wherein the amount of active ingredient is 1 mg - 40 mg.

14. A ready-for-use liquid formulation, comprising an aqueous liquid and the dosage form according to any of claims 1 - 13.

15. The liquid formulation according to claim 14, wherein the amount of the aqueous liquid is from 2. 5 times up to 7.5 times the amount of the suspension modifying granulate (II).

16. The liquid formulation according to any of claims 14 to 15, wherein the aqueous liquid is water.

17. A process for the preparation of the suspension modifying granulate (II) used in the dosage form according to any of claims 1 to 10 wherein the process comprises the step that the rapidly dissolving diluent and the gelling agent are mixed and granulated together and thereafter dried, resulting in that the rapidly dissolving diluent is randomly distributed in and on the obtained individual granule particles.

18. A process for the manufacture of the suspension modifying granulate (II) used in the dosage form as defined in any of claims 1 to 10, wherein the process includes the following steps in the following order;
I) mixing the gelling agent with the pH-regulating agent, the rapidly dissolving diluent, and the optional disintegrant
II) dissolving the binder in ethanol
III) wetting the mixture obtained in step I with the solution obtained in step II
IV) agitating the wet mixture obtained in step III in order to have almost each particle of the gelling agent to be in close/intimate contact with the above mentioned rapidly dissolving diluent
V) drying the agitated wet mixture from step IV until the final moisture content in the suspension modifying granulate (II) measured as loss on drying is < 3 % (w/w),
VI) grinding or milling the dry granules obtained in step V until more than 95% (w/w) of the granules passes a sieve having 1.0 mm openings.

19. A process for the manufacture of the suspension modifying granulate (II) used in the dosage form as defined in any of claims 1 to 10, wherein the process includes the following steps in the following order;
I) dissolving the binder in ethanol
II) mixing the gelling agent with the pH-regulating agent, the rapidly dissolving diluent, and the optional disintegrant
III) wetting the mixture obtained in step II with the solution obtained in step I
IV) agitating the wet mixture obtained in step III in order to have almost each particle of the gelling agent to be in close/intimate contact with the above mentioned rapidly dissolving diluent
V) drying the agitated wet mixture from step IV until the final moisture content in the suspension modifying granulate (II) measured as loss on drying is < 3 % (w/w),
VI) grinding or milling the dry granules obtained in step V until more than 95% (w/w) of the granules passes a sieve having 1.0 mm openings.

20. The pharmaceutical dosage form as defined in any of claims 1-13 for treatment of gastrointestinal diseases.

## Patentansprüche

1. Orale, pharmazeutische Dosierungsform aus einer festen, schnell gelierenden Granulatmischung, die zur Herstellung einer Suspension geeignet ist, die I) einen säureempfindlichen Protonenpumpenhemmer, der Esomeprazol ist, ein alkalisches Salz davon oder eine hydratisierte Form von einem von ihnen als Wirkstoff, der in einer Vielzahl von magensaftresistenten beschichteten Kügelchen verteilt ist und II) ein Granulat umfasst, **dadurch gekennzeichnet, dass** das Granulat ein eine Suspension modifizierendes Granulat ist, welches ein sich schnell auflösendes Verdünnungsmittel, das ausgewählt ist aus Glucose und Saccharose und Hydraten von jedem von ihnen, ein Geliermittel, das unter Xanthangummis ausgewählt ist, ein saures, den pH-Wert regulierendes Mittel, ein Bindemittel und gegebenenfalls ein Zerfallsmittel umfasst und das Granulat frei von Bicarbonatsalzen und/oder Carbonatsalzen ist und wobei das Verhältnis zwischen dem Bindemittel und dem Geliermittel in dem Granulat (II) von 1:2 bis 1:3 (Gew./Gew.) beträgt.

2. Dosierungsform nach Anspruch 1, die frei von Lactose ist.

3. Dosierungsform nach einem der Ansprüche 1 bis 2, wobei das sich schnell auflösende Verdünnungsmittel und das Geliermittel gemischt und zusammen derart granuliert werden, dass das sich schnell auflösende Verdünnungsmittel willkürlich in und an den erhaltenen Granulatteilchen verteilt ist.

4. Dosierungsform nach einem der Ansprüche 1 bis 3, wobei die Konzentration des Geliermittels 0,6 Gew.-% bis 12 Gew.-% von dem die Suspension modifizierenden Granulat (II) beträgt.

5. Dosierungsform nach einem der Ansprüche 1 bis 3, wobei die Konzentration des Geliermittels 1,8 Gew.-% bis 4,8 Gew.-% von dem die Suspension modifizierenden Granulat (II) beträgt.

6. Dosierungsform nach einem der Ansprüche 1 bis 5, wobei das die Suspension modifizierende Granulat (II), wenn es in Wasser suspendiert wird, eine Suspension bildet, die einen pH-Wert in dem Bereich von 3,0 bis 6,0 aufweist.

7. Dosierungsform nach einem der Ansprüche 1 bis 5, wobei das die Suspension modifizierende Granulat (II), wenn es in Wasser suspendiert wird, eine Suspension bildet, die einen pH-Wert in dem Bereich von 3,0 bis 5,0 aufweist.

8. Dosierungsform nach einem der Ansprüche 1 bis 7, wobei die magensaftresistenten beschichteten Kügelchen aus den strukturellen Komponenten bestehen; einem Kernmaterial, welches den Wirkstoff enthält, einer Trennschicht, einer magensaftresistenten Überzugsschicht und keiner zusätzlichen Deckbeschichtung auf der magensaftresistenten Überzugsschicht.

9. Dosierungsform nach einem der Ansprüche 1 bis 8, wobei die magensaftresistenten beschichteten Kügelchen einen durchschnittlichen Durchmesser von 0,2-1,8 mm im Durchmesser aufweisen.

10. Dosierungsform nach einem der Ansprüche 1 bis 8, wobei die magensaftresistenten beschichteten Kügelchen einen durchschnittlichen Durchmesser von 0,4-1,0 mm im Durchmesser aufweisen.

11. Beutel, der die Dosierungsform nach einem der Ansprüche 1 bis 10 umfasst.

12. Beutel nach Anspruch 11, wobei die Menge des Wirkstoffs 1 mg-100 mg beträgt.

13. Beutel nach Anspruch 11, wobei die Menge des Wirkstoffs 1 mg-40 mg beträgt.

14. Gebrauchsfertige flüssige Formulierung, die eine wässrige Flüssigkeit und die Dosierungsform nach einem der Ansprüche 1-13 umfasst.

15. Flüssige Formulierung nach Anspruch 14, wobei die Menge der wässrigen Flüssigkeit das 2,5-Fache bis zu 7,5-Fache der Menge von dem die Suspension modifizierenden Granulat (II) beträgt.

16. Flüssige Formulierung nach einem der Ansprüche 14 bis 15, wobei die wässrige Flüssigkeit Wasser ist.

17. Verfahren zur Präparation des die Suspension modifizierenden Granulats (II), das in der Dosierungsform nach einem der Ansprüche 1 bis 10 eingesetzt wird, wobei das Verfahren den Schritt umfasst, dass das sich schnell auflösende Verdünnungsmittel und das Geliermittel gemischt und zusammen granuliert und anschließend getrocknet werden, was zur Folge hat, dass das sich schnell auflösende Verdünnungsmittel willkürlich in und an den erhaltenen einzelnen Granulatteilchen verteilt ist.

18. Verfahren zur Herstellung des die Suspension modifizierenden Granulats (II), das in der Dosierungsform nach einem der Ansprüche 1 bis 10 eingesetzt wird, wobei das Verfahren die nachfolgenden Schritte in der folgenden Reihenfolge umfasst:
I) Mischen des Geliermittels mit dem den pH-Wert regulierenden Mittel, dem sich schnell auflösenden Verdünnungsmittel und dem optionalen Zerfallsmittel,
II) Auflösen des Bindemittels in Ethanol,
III) Befeuchten der in Schritt I) erhaltenen Mischung mit der Lösung, die in Schritt II) erhalten wurde,
IV) Rühren der in Schritt III) erhaltenen feuchten Mischung, um annähernd jedes Teilchen des Geliermittels in engen/unmittelbaren Kontakt mit dem vorangehend erwähnten, sich schnell auflösenden Verdünnungsmittel zu bringen,
V) Trocknen der gerührten, feuchten Mischung aus Schritt IV), bis der endgültige Feuchtigkeitsgehalt in dem die Suspension modifizierenden Granulat (II), gemessen als Trocknungsverlust, < 3 % (Gew./Gew.) beträgt,
VI) Zerreiben oder Mahlen des trockenen Granulats, das in Schritt V) erhalten wurde, bis mehr als 95 % (Gew./Gew.) des Granulats durch ein Sieb gelaufen ist, das Öffnungen von 1,0 mm aufweist.

19. Verfahren zur Herstellung des die Suspension modifizierenden Granulats (II), das in der Dosierungsform nach einem der Ansprüche 1 bis 10 eingesetzt wird, wobei das Verfahren die nachfolgenden Schritte in der folgenden Reihenfolge umfasst:
I) Auflösen des Bindemittels in Ethanol,
II) Mischen des Geliermittels mit dem den pH-Wert regulierenden Mittel, dem sich schnell auflösenden Verdünnungsmittel und dem optionalen Zerfallsmittel,
III) Befeuchten der in Schritt II) erhaltenen Mischung mit der Lösung, die in Schritt I) erhalten wurde,
IV) Rühren der in Schritt III) erhaltenen feuchten Mischung, um annähernd jedes Teilchen des Geliermittels in engen/unmittelbaren Kontakt mit dem vorangehend erwähnten, sich schnell auflösenden Verdünnungsmittel zu bringen,
V) Trocknen der gerührten, feuchten Mischung aus Schritt IV), bis der endgültige Feuchtigkeitsgehalt in dem die Suspension modifizierenden Granulat (II), gemessen als Trocknungsverlust, < 3 % (Gew./Gew.) beträgt,
VI) Zerreiben oder Mahlen des trockenen Granulats, das in Schritt V) erhalten wurde, bis mehr als 95 % (Gew./Gew.) des Granulats durch ein Sieb gelaufen ist, das Öffnungen von 1,0 mm aufweist.

20. Pharmazeutische Dosierungsform nach einem der Ansprüche 1-13 zur Behandlung von Erkrankungen des Magen-Darm-Traktes.

## Revendications

1. Forme galénique pharmaceutique orale qui est un mélange granulé solide à gélification rapide adapté à la fabrication d'une suspension, et qui comprend I) un inhibiteur de la pompe à protons sensible acide qui est l'ésoméprazole, l'un de ses sels alcalins ou une forme hydratée du composé ou de l'un quelconque de ses sels au titre de principe actif, répartis en une multitude de granules à enrobage entérique et II) un granulat, **caractérisé en ce que** le granulat est un granulat modificateur de suspension qui comprend un diluant à dissolution rapide choisi parmi le glucose et le sucrose ainsi que leurs hydrates, un agent gélifiant choisi parmi les gommes xanthane, un agent de régulation du pH acide, un agent liant et un agent délitant optionnel, **en ce que** le granulat ne contient pas de sels de bicarbonate et/ou de sels de carbonate, et **en ce que** le rapport massique entre l'agent liant et l'agent gélifiant dans le granulat (II) est compris entre 1:2 et 1:3.

2. Forme galénique conforme à la revendication 1, et ne comprenant pas de lactose.

3. Forme galénique conforme à l'une quelconque des revendications 1 à 2, où le diluant à dissolution rapide et l'agent gélifiant sont mélangés et granulés ensemble de sorte que le diluant à dissolution rapide soit distribué de façon aléatoire à l'intérieur et à la surface des particules granulées obtenues.

4. Forme galénique conforme à l'une quelconque des revendications 1 à 3, où la concentration de l'agent gélifiant est comprise entre 0,6 % et 12 % en masse du granulat modificateur de suspension (II).

5. Forme galénique conforme à l'une quelconque des revendications 1 à 3, où la concentration de l'agent gélifiant est comprise entre 1,8 % et 4,8 % en masse du granulat modificateur de suspension (II).

6. Forme galénique conforme à l'une quelconque des revendications 1 à 5, où le granulat modificateur de suspension (II), lorsqu'il est dispersé dans l'eau, forme une suspension dont le pH est compris dans l'intervalle 3,0 à 6,0.

7. Forme galénique conforme à l'une quelconque des revendications 1 à 5, où le granulat modificateur de suspension (II), lorsqu'il est dispersé dans l'eau, forme une suspension dont le pH est compris dans l'intervalle 3,0 à 5,0.

8. Forme galénique conforme à l'une quelconque des revendications 1 à 7, où les granules à enrobage entérique sont constitués des composants structurels suivants : une matière de noyau comprenant le principe actif, une couche de sous-enrobage, une couche d'enrobage entérique et aucune couche d'enrobage supplémentaire par-dessus l'enrobage entérique.

9. Forme galénique conforme à l'une quelconque des revendications 1 à 8, où les granules à enrobage entérique présentent un diamètre moyen compris entre 0,2 et 1,8 mm.

10. Forme galénique conforme à l'une quelconque des revendications 1 à 8, où les granules à enrobage entérique présentent un diamètre moyen compris entre 0,4 et 1,0 mm.

11. Sachet comprenant la forme galénique conforme à l'une quelconque des revendications 1 à 10.

12. Sachet conforme à la revendication 11, où la quantité de principe actif est comprise entre 1 mg et 100 mg.

13. Sachet conforme à la revendication 11, où la quantité de principe actif est comprise entre 1 mg et 40 mg.

14. Formule liquide prête à l'emploi, comprenant un liquide aqueux et la forme galénique conforme à l'une quelconque des revendications 1 à 13.

15. Formule liquide conforme à la revendication 14, où la quantité de liquide aqueux est entre 2,5 et 7,5 fois supérieure à la quantité du granulat modificateur de suspension (II).

16. Formule liquide conforme à l'une quelconque des revendications 14 à 15, où le liquide aqueux est l'eau.

17. Procédé d'élaboration du granulat modificateur de suspension (II) employé dans la forme galénique conforme à l'une quelconque des revendications 1 à 10, le procédé comprenant l'étape de mélangeage et de granulation simultanés du diluant à dissolution rapide et de l'agent gélifiant, qui sont ensuite séchés, ladite étape résultant en la distribution aléatoire du diluant à dissolution rapide à l'intérieur et en surface de chacune des particules granulées obtenues.

18. Procédé de fabrication du granulat modificateur de suspension (II) employé dans la forme galénique conforme à l'une quelconque des revendications 1 à 10, le procédé incluant les étapes suivantes dans l'ordre suivant :
I) mélangeage de l'agent gélifiant à l'agent régulateur de pH, au diluant à dissolution rapide et à l'agent délitant optionnel
II) dissolution de l'agent liant dans l'éthanol
III) mouillage du mélange obtenu dans l'étape I par la solution obtenue dans l'étape II
IV) agitation du mélange humide obtenu dans l'étape III pour obtenir un contact proche/intime de la quasi-totalité des particules d'agent gélifiant avec le diluant à dissolution rapide susmentionné
V) séchage du mélange humide agité de l'étape IV jusqu'à ce que la teneur finale en humidité dans le granulat modificateur de suspension (II), mesurée par perte au séchage, soit inférieure à 3 % en masse,
VI) broyage ou fraisage des granules secs obtenus dans l'étape V jusqu'à ce que plus de 95 % en masse des granules passent à travers un tamis de maillage 1,0 mm.

19. Procédé de fabrication du granulat modificateur de suspension (II) employé dans la forme galénique conforme à l'une quelconque des revendications 1 à 10, le procédé incluant les étapes suivantes dans l'ordre suivant :
I) dissolution de l'agent liant dans l'éthanol
II) mélangeage de l'agent gélifiant à l'agent régulateur de pH, au diluant à dissolution rapide et à l'agent délitant optionnel
III) mouillage du mélange obtenu dans l'étape II par la solution obtenue dans l'étape I
IV) agitation du mélange humide obtenu dans l'étape III pour obtenir un contact proche/intime de la quasi-totalité des particules d'agent gélifiant avec le diluant à dissolution rapide susmentionné
V) séchage du mélange humide agité de l'étape IV jusqu'à ce que la teneur finale en humidité dans le granulat modificateur de suspension (II), mesurée par perte au séchage, soit inférieure à 3 % en masse,
VI) broyage ou fraisage des granules secs obtenus dans l'étape V jusqu'à ce que plus de 95 % en masse des granules passent à travers un tamis de maillage 1,0 mm.

20. Forme galénique pharmaceutique conforme à l'une quelconque des revendications 1 à 13, destinée au traitement des maladies gastro-intestinales.
